# EUROPEAN PATENT SPECIFICATION

(11) **EP 3 856 092 B1**
(45) Date of publication and mention of the grant of the patent: **18.12.2024**
(21) Application number: 19804893.6
(22) Date of filing: 25.09.2019
(51) Int. Cl.: A61F 2/95

(54) **CONSTRAINING SYSTEMS**
EINSCHRÄNKUNGSSYSTEME
SYSTÈMES DE CONTRAINTE

(30) Priority: 26.09.2018 US 201862737040 P; 05.10.2018 US 201862741937 P
(43) Date of publication of application: 04.08.2021
(73) Proprietor: W. L. Gore & Associates, Inc., Newark, DE 19711 (US)
(72) Inventor: RAMIREZ, Gil R., Newark, Delaware 19711 (US); STASTKA, Jerry J., Newark, Delaware 19711 (US); TENNANT, John S., Newark, Delaware 19711 (US); YAMAMOTO, Marc Y., Newark, Delaware 19711 (US)
(74) Representative: HGF
(86) International application number: PCT/US2019/052921
(87) International publication number: WO 2020/068957

(56) References cited:
- EP-A1- 1 087 726

## Description

### FIELD

The present disclosure relates generally to apparatuses, systems, and methods that include coverings used in delivery of implantable medical devices. More specifically, the present disclosure relates to apparatuses, systems, and methods that include coverings for constraining expandable, implantable medical devices during device delivery.

### BACKGROUND

Stents and stent-grafts may be utilized to radially support a variety of tubular passages in the body, including arteries, veins, airways, gastrointestinal tracts, and biliary tracts. The preferred method of placing these devices has been to use specialized delivery systems to precisely place and deploy a device at the site to be treated. These delivery systems allow the practitioner to minimize the trauma and technical difficulties associated with device placements. Attributes of delivery systems include: low profile; ability to pass through introducer sheaths; ability to negotiate tortuous vasculature smoothly and atraumatically; protection of constrained devices; and ability to accurately position and deploy the device.

Stents or stent-grafts may be deployed and plastically deformed by using an inflatable balloon (*e*.*g*., balloon expandable stents) or to self-expand and elastically recover (*e*.*g*., self-expandable stents) from a collapsed or constrained delivery diameter to an expanded and deployed diameter.

These stent and stent-graft devices may be held, compressed, or constrained in the delivery configuration prior to and during delivery to a target location.

EP1087726 A1 describes a thin tubular multiple filament (film or fiber) structure that can hold high internal pressures. When desired, an extension of the filaments can be pulled in any direction to unfurl the structure. This device is useful for self expanding stent or stent graft delivery systems, balloon dilatation catheters, removable guide wire lumens for catheters, drug infusion or suction catheters, guide wire bundling casings, removable filters, removable wire insulation, removable packaging and other applications.

### SUMMARY

The invention relates to a delivery system as described according to the appended claims.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is a top plan view of a delivery system including a catheter with a removable constraint, in accordance with an embodiment;
FIG. 2 is a side view of an implantable medical device including a removable constraint, in accordance with an embodiment;
FIG. 3 is a schematic view of interlocking strands, in accordance with an embodiment;
FIG. 4 is an end view of the removable constraint showing example knot rows, in accordance with an embodiment;
Fig. 5 is an end view of the removable constraint showing example knot rows, in accordance with an embodiment;
FIGS. 6A-6B are images of a delivery system in a delivery configuration and a semi-deployed configuration, respectively, in accordance with an embodiment;
FIG. 7 is a graph comparing knit force for deployment of an implantable medical device.

As the terms are used herein with respect to ranges of measurements "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error, differences in measurement and/or manufacturing equipment calibration, human error in reading and/or setting measurements, adjustments made to optimize performance and/or structural parameters in view of differences in measurements associated with other components, particular implementation scenarios, imprecise adjustment and/or manipulation of objects by a person or machine, and/or the like.

While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### DETAILED DESCRIPTION

Persons skilled in the art will readily appreciate that various aspects of the present disclosure can be realized by any number of methods and apparatus configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward apparatuses, systems, and methods that include forming or manufacturing a constraining mechanism. The constraining mechanisms are configured to hold, compress, or constrain an implantable medical device (*e*.*g*., a stent, stent-graft, balloon, or other expandable medical device) in a delivery configuration prior to and during delivery to a target location. In certain instances, the constraining mechanism includes one or more fibers.

The constraining mechanisms, in accordance with the various aspects of the present disclosure, may be formed or manufactured directly on the implantable medical device. The fiber or fibers are knit, sewn, or interlocked to form the constraining mechanisms. Thus, the fiber or fibers are knit, sewn, or interlocked together about or around the implantable medical device. As noted above, the implantable medical devices are reduced, collapsed, or constrained to a reduced (delivery) diameter by the constraining mechanism for delivery into a target location into a patient where it is deployed or expanded to a deployed diameter (larger than the reduced diameter).

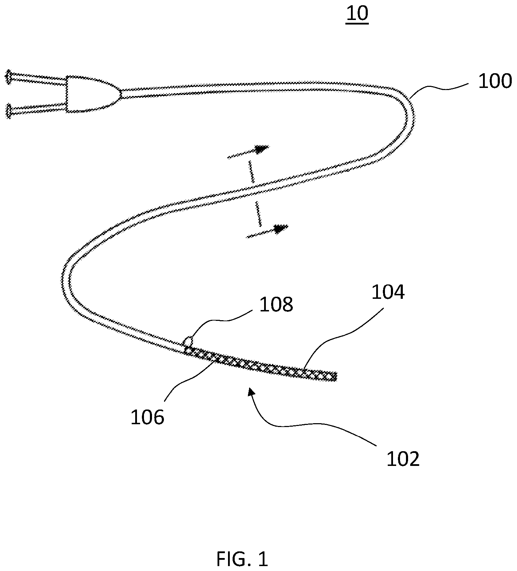

FIG. 1 is a top plan view of a delivery system 10 including a catheter 100 with a removable constraint 102, according to some embodiments. As shown in FIG. 1, the removable constraint 102 is configured to constrain an implantable medical device 104 to a delivery configuration. The removable constraint102 may include one or more fibers 106 arranged about the device 104 to maintain the removable constraint 102 in a constrained configuration.

The removable constraint 102 is arranged along a length of the device 104. The removable constraint 102 is also circumferentially arranged about the device 104 and may substantially cover the device 104 for delivery. The one or more fibers 106 may be arranged within a lumen (not shown) of the catheter 100 and extend toward a proximal end of the catheter 100 that is arranged external to a patient during delivery of the device 104. The one or more fibers 106 include a proximal end 108 that a user may apply tension to in order to release the removable constraint 102 and deploy the device 104.

In certain instances, the one or more fibers 106 release similar to a rip cord such that interlocking portions (*e*.*g*., overlapping fibers or knots) sequentially release along the length of the device 104. As is explained in greater detail below, the removable constraint 102 is formed by interlocking together the one or more fibers 106 directly on the device 104. The device 104 may be a stent, stent-graft, a balloon, or a similar device.

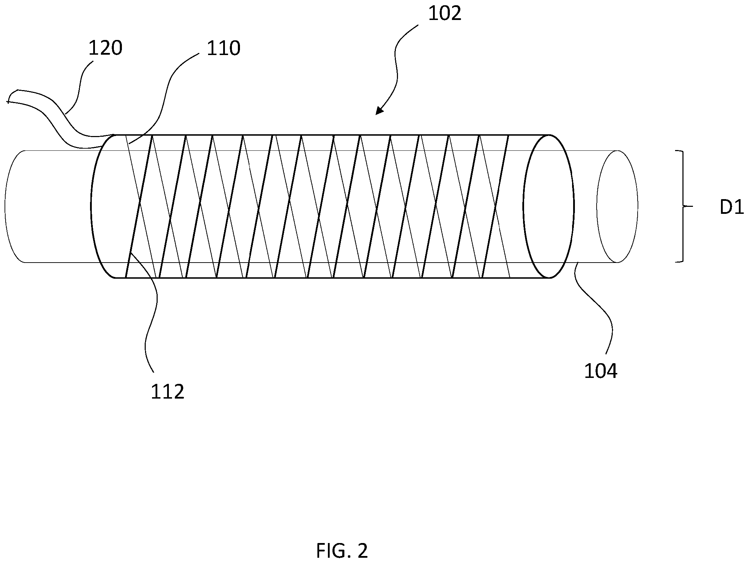

FIG. 2 is a side view of the device 104 including the removable constraint 102, in accordance with an embodiment. As shown, the device 104 includes a delivery diameter D1 and a deployed diameter D2 (not shown) that is larger than the delivery diameter D1. The removable constraint 102 is arranged about the device 104 at the delivery diameter D1. As shown, the removable constraint 102 includes at least two interlocking strands in the form of a warp knit. For example, the removable constraint 102 may include a first interlocking strand 110 and a second interlocking strand 112. The first and/or the second interlocking strand(s) 110, 112 may operate, for example, as a deployment line 120 configured to release the removable constraint 102 and release the device 104 from the delivery diameter D1 to the deployed diameter D2 in response to a knit force applied to the deployment line 120.

The device 104 may have a desired deployed diameter D2 from about 5mm-15mm, or 6mm-9mm, or 6mm-12mm, for example, and a delivery diameter D1 that is less than the deployed diameter D2. For example, in some instances, a ratio of the delivery diameter D1 of the device 104 to the deployed diameter D2 (not shown) of the device 104 is less than about 0.3, less than about 0.29, less than about 0.28, less than about 0.27, or less than about 0.26.

In some instances, the first interlocking strand 110 has at least one strand property that is different than the second interlocking strand 112, which may affect the amount of knit force required to release the removable constraint 102. Examples of differing strand properties can include strand thickness, strand denier, strand coefficient of friction, strand material, and/or strand stiffness.

The first interlocking strand 110, for example, may have a first diameter or thickness and the second interlocking strand 112 may have a second diameter or thickness that is greater than the first diameter. In various examples, use of a larger diameter or thickness for the second interlocking strand 112 helps increase friction between the first and second interlocking strands 110, 112, in turn helping maintain the device 104 in the delivery configuration. In some instances where the differing strand property is strand diameter or thickness, the first and second interlocking strands 110, 112 may have a cross-sectional diameter or thickness that varies by about 0.0015 cm (0.0006 inches). In other examples, the cross sectional diameter may vary by about 0.0008 cm, 0.0009 cm, 0.0010 cm, 0.0011 cm, 0.0012 cm, 0.0013 cm, 0.0014 cm, 0.0015 cm, 0.0016 cm, 0.0017 cm, 0.0018 cm, 0.0019 cm, 0.0020 cm, 0.0021 cm, 0.0022 cm, or any number therebetween. In addition, the difference in diameter between the first and second interlocking strands 110, 122 may be between about 0.00127 cm (0.0005 inches) and about 0.0203 cm (0.008 inches). For reference, the term "diameter" is not meant to require a circular cross-section, and is instead to be understood broadly to reference a maximum transverse cross-sectional dimension of a strand or effective diameter.

For reference, the term "diameter" is not meant to require a circular cross-section, and is instead to be understood broadly to reference a maximum transverse cross-sectional dimension of a strand.

In various examples, the first interlocking strand 110 may include a first material while the second interlocking strand 112 may include a second material that is different than the first material (e.g., either as an alternative to differing diameters between strands or as an additional feature). The first interlocking strand 110 may include a material having a different (*e*.*g*., higher or lower) tensile strength as compared to a tensile strength of a material of the second interlocking strand 112 in certain instances. Additionally or alternatively, the first interlocking strand 110 may include a material having a different (*e*.*g*., higher or lower) surface roughness as compared to a surface roughness of a material of the second interlocking strand 112. Further, the first interlocking strand 110 may include a material having a different (*e*.*g*., higher or lower) tackiness or adherence to other materials as compared to a tackiness or adherence of a material of the second interlocking strand 112. In various examples, the first and second strands 110, 112 exhibit different coefficients of static and/or kinetic friction, with the second strand 112 exhibiting a relatively higher coefficient of static and/or kinetic friction than the first strand 110, for example. From the foregoing, it should be appreciated that the strands 110, 112 may include multiple different properties. For example, the first interlocking strand 110 may have a different (*e*.*g*., higher or lower) tensile strength and a different (*e*.*g*., higher or lower) surface roughness as compared to the second interlocking strand 112.

Potential materials for strands 110, 112 discussed herein include, for example, polytetrafluoroethylene (PTFE), expanded polytetrafluoroethylene (ePTFE), polyester, polyurethane, fluoropolymers, such as perfluoroelastomers and the like, polytetrafluoroethylene, silicones, urethanes, ultra-high molecular weight polyethylene, aramid fibers, and combinations thereof. Other embodiments for strands 110, 112 can include high strength polymer fibers such as ultra-high molecular weight polyethylene fibers (e.g., Spectra^{®}, Dyneema Purity^{®}, etc.) or aramid fibers (e.g., Technora^{®}, etc.). Generally, any of the foregoing properties may be assessed using ASTM or other recognized measurement techniques and standards, as would be appreciated by a person of ordinary skill in the field.

In certain instances, the first and second interlocking strands 110, 112 may include the same material but may differ in other aspects. For example, one of the strands may include fillers or core materials, may be surface treated by etching, vapor deposition, or coronal or other plasma treatment, among other treatment types, including being coated with suitable coating materials. Similar to the differing diameter, use of differing strand materials for the strands 110, 112 may increase friction between the first and second interlocking strands 110, 112 to help maintain the device 104 in the delivery configuration.

The device 104 has a radial force at the delivery diameter D1. The radial force generally refers to the force caused by the device 104 acting on the removable constraint 102 at any point during deployment of the device 104. As discussed above, the interlocking strands 110, 112 are adapted to be removed with a deployment force applied to the deployment line 120. In some instances, the ratio of this radial force of the device 104 to the knit force applied to the deployment line 120 is less than about 500. In other instances, the ratio of this radial force of the device 104 to the knit force applied to the deployment line 120 is less than about 475. In addition, the ratio of this radial force of the device 104 to the knit force applied to the deployment line 120 may be less than about 450. In addition, the ratio of this radial force of the device 104 to the knit force applied to the deployment line 120 is less than about 425 in other instances. Further, the ratio of the radial force to the knit force may be between about 10, 20, 30, 40, 50, 100, 200, 300, 400 (or any number in between) and about 500, between about 10, 20, 30, 40, 50, 100, 200, 300, 400 (or any number in between) and about 475, between about 10, 20, 30, 40, 50, 100, 200, 300, 400 (or any number in between) and about 450, or between about 10, 20, 30, 40, 50, 100, 200, 300, 400 (or any number in between) and about 425, for example.

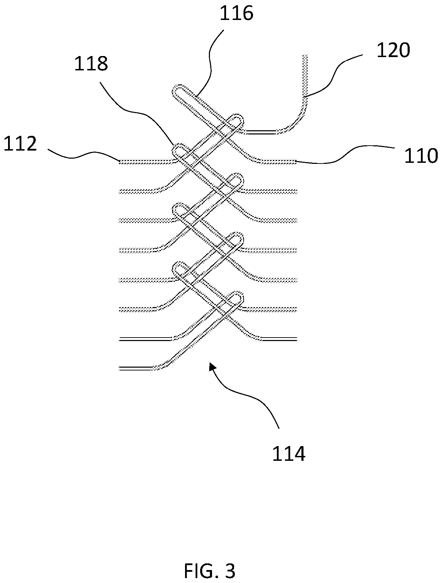

FIG. 3 is a schematic view of interlocking strands of the removable constraint 102 (FIGS. 1-2), in accordance with an embodiment. The interlocking strands (e.g., the first and second interlocking strands 110, 112, as shown) are generally interwoven with one another to form at least one knot row 114. As shown, the knot row 114 is formed of interlocking loops formed from the first and second interlocking strands 110, 112. For example, first interlocking loops 116 are formed by the first interlocking strand 110 and are interwoven with second interlocking loops 118 formed by the second interlocking strand 112. This interlocking, looped configuration allows for release of the removable constraint 102 (FIGS. 1-2) by applying the deployment force (releases knit force) to the deployment line 120.

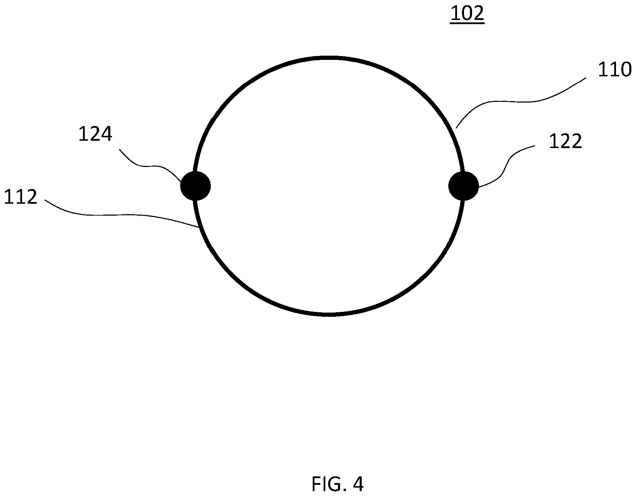

In general, the knot row 114 can be positioned at any location about the circumference of the removable constraint 102. In certain instances, the interlocking strands may form more than one knot row. FIG. 4 is an end view of the removable constraint 102 showing example knot rows 114, in accordance with an embodiment. As shown, the first and second interlocking strands 110, 112 may form a first knot row 122 and a second knot row 124 positioned at different locations about the circumference of the removable constraint 102. For example, the knot rows can be spaced approximately 180 degrees apart from one another, approximately 90 degrees apart from one another, approximately 60 degrees apart from one another, or any other distance as desired.

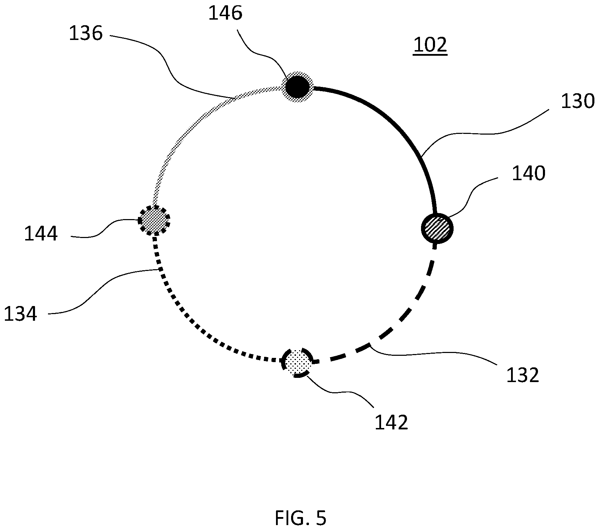

FIG. 5 is an end view of the removable constraint 102 showing example knot rows 114, in accordance with an embodiment. As shown, in some instances, the removable constraint 102 may include more than two interlocking strands. For example, the removable constraint 102 may include a first interlocking strand 130, a second interlocking strand 132, a third interlocking strand 134, and a fourth interlocking strand 136, for example. In these instances, the removable constraint 102 can also include a first knot row 140, a second knot row 142, a third knot row 144, and a fourth knot row 146 corresponding with the first through fourth interlocking strands 130-136, respectively. Applying a force to one of the first through fourth interlocking strands 130-136 may initiate unravel of the corresponding knot row, thus allowing for selective deployment of various portions of the removable constraint 102.

In some instances, at least one of the interlocking strands may have a different strand property than the remaining interlocking strands. For example, the first, second, and third strands 130, 132, 134 may have the same strand property, while the fourth strand 136 has a different strand property. In other examples, two of the interlocking strands may have one strand property while the remaining strands have a second strand property that is different from the first strand property, and so on. In some examples, each of the interlocking strands may have a different strand property to facilitate selective deployment of various portions of the removable constraint 102 as desired. In certain instances, one of the interlocking strands 130, 132, 134, 136 has a different property than an adjacent one of the interlocking strands 130, 132, 134, 136.

As discussed above, the knot rows can be positioned at any location about the circumference of the removable constraint 102. Though shown in FIG. 5 as spaced approximately 90 degrees apart from one another, the knot rows can be spaced any amount as desired. For example, the knot rows can be spaced 60 degrees apart from one another, 45 degrees apart from one another, or less as desired to achieve the desired deployment length ratio.

In some instances, having interlocking strands with differing properties can lessen ramping of the device 104 prior to being released. For example, the interlocking strands may lessen ramping (deployment angle) of the device 104 prior to the knots of the knot row 114 being released in sequence. Ramping of the device 104 may lead to advanced or pre-deployment of the device 104 prior to an intended deployment.

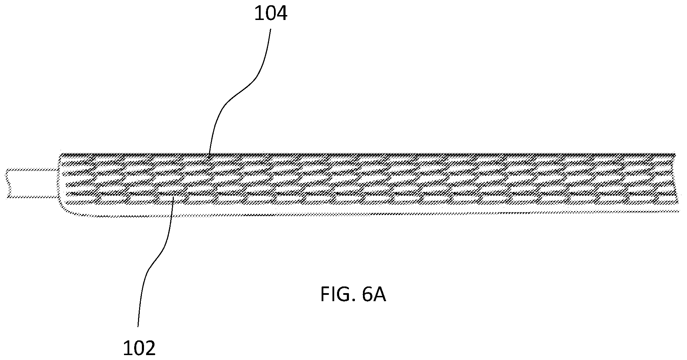

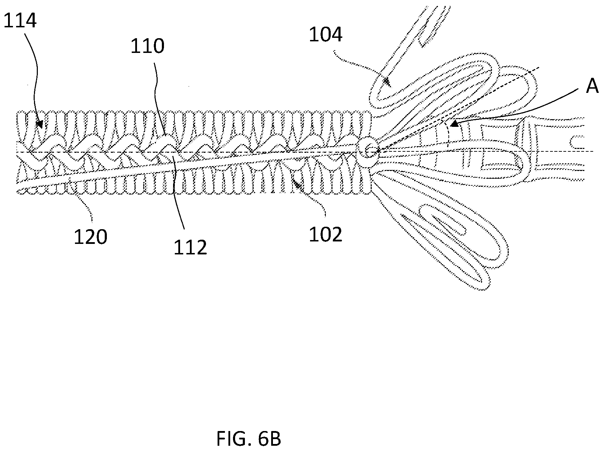

FIGS. 6A-6B are images of a delivery system in a delivery configuration and a semi-deployed configuration, respectively, in accordance with an embodiment. As shown in FIG. 6A, the removable constraint 102 is attached to the device 104 at its delivery diameter D1. During deployment, a knit force is applied to the deployment line 120 to release the removable constraint 102 by unraveling the knot row 114, as shown in FIG. 5B. The device 104 is released to the deployed diameter D2 as the removable constraint 102 is released.

The interlocking strands 110, 112 with differing properties can lessen ramping of the device 104 prior to being released. For example, the interlocking strands 110, 112 may lessen ramping (or deployment angle) of the device 104 prior to the knots of the knot row 114 being released in sequence. The device 104 begins to expand to a larger diameter after release of the constraining mechanism 102. The device 104 may be have an angle A between the portions held by the constraining mechanism 102 and portions that have been expanded or are beginning to expand. Due to the angle A and the device 104 expending a force to deploy to the deployed diameter D2, prior constrains may shift due to ramping or expansion of a portion of the device 104. The differing strand properties, however, can lessen ramping of the device 104 by maintaining a location of each of the knots, relative to the device 104, as the knots are released in sequence, lessening undesired deployment (e.g., accelerated deployment or pre-deployment) of the device 104 as described in detail above.

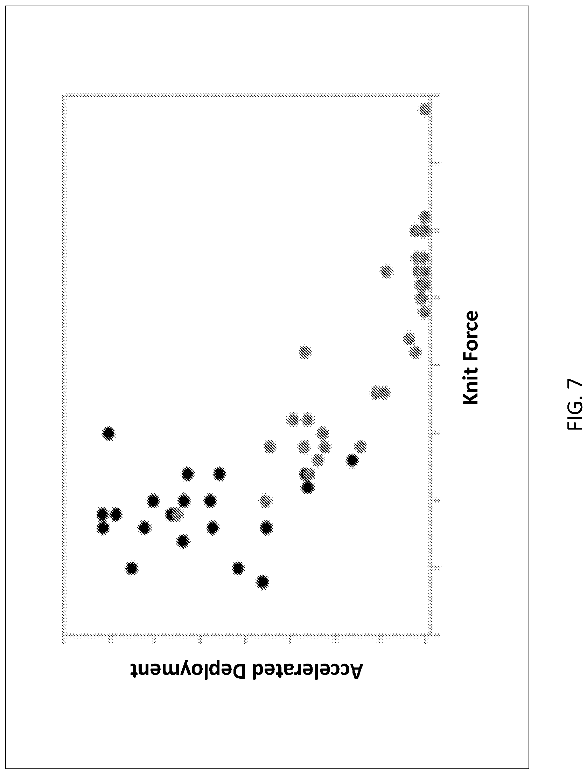

FIG. 7 is a graph comparing knit force for deployment of an implantable medical device as discussed herein. The knit force is the force applied to deployment line to remove a knit loop from within an encompassing knit loop and accelerated deployment. As shown in FIG. 7, as knit force increases, accelerated deployment (AD) decreases. Described herein is a way of achieving increased knit force. This may be evident when constrained diameter to expanded diameter ratio is less than 0.3 or with larger nominal diameter devices (e.g., 9mm, 10mm, 11mm, 12mm, 13mm, 14mm, 15mm, 16mm, and larger including 25mm, 30mm, 40mm, and 50mm).

## Claims

1. A delivery system (10) comprising:
an implantable medical device (104); and
a removable constraint (102) arranged about and configured to releasably constrain the implantable medical device (104), the removable constraint (102) including at least two interlocking strands including a first interlocking strand (110) having a different strand property than a second interlocking strand (112), wherein the first interlocking strand (110) comprises a deployment line (120) configured to release the removeable constraint (102) in response to a force applied to the deployment line (120), **characterized in that** the different strand property comprises strand thickness.

2. The delivery system (10) of claim 1, wherein a ratio of implantable medical device (104) delivery diameter to deployment diameter is less than 0.3.

3. The delivery system (10) of any one of claims 1-2, wherein the differing strand property comprises strand denier.

4. The delivery system (10) of any one of claims 1-2, wherein the differing strand property comprises strand coefficient of friction.

5. The delivery system (10) of any one of claims 1-2, wherein the differing strand property comprises strand material.

6. The delivery system (10) of any one of claims 1-2, wherein the differing strand property comprises strand stiffness.

7. The delivery system (10) of any one of claims 1-6, wherein the implantable medical device (104) has a radial force at a delivery diameter of the implantable medical device (104), and wherein the at least two interlocking strands are adapted to be removed with a knit force applied to the deployment line (120); and wherein a ratio of the radial force to the knit force is: between about 100 and about 500; between about 170 and about 475, between about 225 and about 450; or between about 200 and about 425.

8. The delivery system of claim 1, wherein the at least two interlocking strands are in the form of a warp knit.

9. The delivery system of claim 8, wherein the implantable medical device (104) has a delivery diameter, a deployed diameter, and a radial force at the delivery diameter, and
the removable constraint (102) is attached to the implantable medical device at its delivery diameter, and the removable constraint is configured to be remotely removed when a deployment force is applied to the deployment line; and
wherein a ratio of the radial force to the deployment force is between about 150 and about 500

10. The delivery system of claim 9, wherein the differing strand property further comprises at least one of strand denier, strand coefficient of friction, strand material, and strand stiffness; and optionally
wherein a difference between a cross-sectional diameter of the first strand and a cross-sectional diameter of a second strand is about 0.0161 cm² (0.0025 square inches), or
wherein the strand thickness of the first strand and the second strand varies by between about 0.00127 cm (0.0005 inches) and about 0.0203 cm (0.008 inches).

11. The expandable medical device of any one of claims 9 to 10, wherein the ratio of the radial force to the knit force is below about 450.

## Patentansprüche

1. Abgabesystem (10), umfassend:
eine implantierbare medizinische Vorrichtung (104); und
eine entfernbare Einschränkung (102), die um die implantierbare medizinische Vorrichtung (104) angeordnet ist und dazu konfiguriert ist, diese lösbar einzuschränken, wobei die entfernbare Einschränkung (102) mindestens zwei ineinandergreifende Stränge einschließt, einschließlich eines ersten ineinandergreifenden Strangs (110), der eine unterschiedliche Strangeigenschaft aufweist als ein zweiter ineinandergreifender Strang (112), wobei der erste ineinandergreifende Strang (110) eine Entfaltungsleine (120) umfasst, die dazu konfiguriert ist, die entfernbare Einschränkung (102) als Reaktion auf eine auf die Entfaltungsleine (120) ausgeübte Kraft zu lösen, **dadurch gekennzeichnet, dass** die unterschiedliche Strangeigenschaft Strangdicke umfasst.

2. Abgabesystem (10) nach Anspruch 1, wobei ein Verhältnis eines Abgabedurchmessers der implantierbaren medizinischen Vorrichtung (104) zu einem Entfaltungsdurchmesser kleiner als 0,3 ist.

3. Abgabesystem (10) nach einem der Ansprüche 1-2, wobei die unterschiedliche Strangeigenschaft Strangdenier umfasst.

4. Abgabesystem (10) nach einem der Ansprüche 1-2, wobei die unterschiedliche Strangeigenschaft Strangreibungskoeffizient umfasst.

5. Abgabesystem (10) nach einem der Ansprüche 1-2, wobei die unterschiedliche Strangeigenschaft Strangmaterial umfasst.

6. Abgabesystem (10) nach einem der Ansprüche 1-2, wobei die unterschiedliche Strangeigenschaft Strangsteifigkeit umfasst.

7. Abgabesystem (10) nach einem der Ansprüche 1-6, wobei die implantierbare medizinische Vorrichtung (104) eine radiale Kraft an einem Abgabedurchmesser der implantierbaren medizinischen Vorrichtung (104) aufweist, und wobei die mindestens zwei ineinandergreifenden Stränge dazu angepasst sind, mit einer auf die Entfaltungsleine (120) ausgeübten Strickkraft entfernt zu werden; und wobei ein Verhältnis der radialen Kraft zur Strickkraft Folgendes beträgt: zwischen etwa 100 und etwa 500; zwischen etwa 170 und etwa 475, zwischen etwa 225 und etwa 450; oder zwischen etwa 200 und etwa 425.

8. Abgabesystem nach Anspruch 1, wobei die mindestens zwei ineinandergreifenden Stränge die Form eines Kettengewirkes haben.

9. Abgabesystem nach Anspruch 8, wobei die implantierbare medizinische Vorrichtung (104) einen Abgabedurchmesser, einen entfalteten Durchmesser und eine radiale Kraft am Abgabedurchmesser aufweist, und
die entfernbare Einschränkung (102) an der implantierbaren medizinischen Vorrichtung an ihrem Abgabedurchmesser befestigt ist, und die entfernbare Einschränkung dazu konfiguriert ist, aus der Ferne entfernt zu werden, wenn eine Entfaltungskraft auf die Entfaltungsleine ausgeübt wird; und
wobei ein Verhältnis der Radialkraft zur Entfaltungskraft zwischen etwa 150 und etwa 500 liegt.

10. Abgabesystem nach Anspruch 9, wobei die unterschiedliche Strangeigenschaft ferner mindestens eine von Strangdenier, Strangreibungskoeffizient, Strangmaterial und Strangsteifigkeit umfasst; und optional
wobei ein Unterschied zwischen einem Querschnittsdurchmesser des ersten Strangs und einem Querschnittsdurchmesser eines zweiten Strangs etwa 0,0161 cm² (0,0025 Quadratzoll) beträgt, oder
wobei die Strangdicke des ersten Strangs und des zweiten Strangs um zwischen etwa 0,00127 cm (0,0005 Zoll) und etwa 0,0203 cm (0,008 Zoll) variiert.

11. Expandierbare medizinische Vorrichtung nach einem der Ansprüche 9 bis 10, wobei das Verhältnis der Radialkraft zur Strickkraft unter etwa 450 liegt.

## Revendications

1. Système de pose (10) comprenant :
un dispositif médical implantable (104) ; et
une contrainte amovible (102) disposée autour et configurée pour contraindre de manière amovible le dispositif médical implantable (104), la contrainte amovible (102) comprenant au moins deux brins entrelacés comprenant un premier brin entrelacé (110) comportant une propriété de brin différente de celle d'un second brin entrelacé (112), dans lequel le premier brin entrelacé (110) comprend une ligne de déploiement (120) configurée pour libérer la contrainte amovible (102) en réponse à une force appliquée à la ligne de déploiement (120), **caractérisé en ce que** la propriété de brin différente comprend l'épaisseur du brin.

2. Système de pose (10) selon la revendication 1, dans lequel un rapport entre le diamètre de pose du dispositif médical implantable (104) et le diamètre de déploiement est inférieur à 0,3.

3. Système de pose (10) selon l'une quelconque des revendications 1-2, dans lequel la propriété de brin différente comprend le denier du brin.

4. Système de pose (10) selon l'une quelconque des revendications 1-2, dans lequel la propriété de brin différente comprend le coefficient de frottement du brin.

5. Système de pose (10) selon l'une quelconque des revendications 1-2, dans lequel la propriété de brin différente comprend le matériau du brin.

6. Système de pose (10) selon l'une quelconque des revendications 1-2, dans lequel la propriété de brin différente comprend la rigidité du brin.

7. Système de pose (10) selon l'une quelconque des revendications 1-6, dans lequel le dispositif médical implantable (104) comporte une force radiale au niveau d'un diamètre de pose du dispositif médical implantable (104), et dans lequel les au moins deux brins entrelacés sont conçus pour être retirés avec une force de tricotage appliquée à la ligne de déploiement (120) ; et dans lequel un rapport de la force radiale à la force de tricotage est : entre environ 100 et environ 500 ; entre environ 170 et environ 475, entre environ 225 et environ 450 ; ou entre environ 200 et environ 425.

8. Système de pose selon la revendication 1, dans lequel les au moins deux brins entrelacés se présentent sous la forme d'un tricot chaîne.

9. Système de pose selon la revendication 8, dans lequel le dispositif médical implantable (104) comporte un diamètre de pose, un diamètre déployé et une force radiale au niveau du diamètre de pose, et
la contrainte amovible (102) est fixée au dispositif médical implantable au niveau de son diamètre de pose, et la contrainte amovible est configurée pour être retirée à distance lorsqu'une force de déploiement est appliquée à la ligne de déploiement ; et
dans lequel un rapport entre la force radiale et la force de déploiement est compris entre environ 150 et environ 500.

10. Système de pose selon la revendication 9, dans lequel la propriété de brin différente comprend en outre au moins parmi le denier du brin, le coefficient de frottement du brin, le matériau du brin et la rigidité du brin ; et éventuellement
dans lequel une différence entre un diamètre de section transversale du premier brin et un diamètre de section transversale d'un second brin est d'environ 0,0161 cm² (0,0025 pouce carré), ou
dans lequel l'épaisseur du brin du premier brin et du second brin varie entre environ 0,00127 cm (0,0005 pouce) et environ 0,0203 cm (0,008 pouce).

11. Dispositif médical extensible selon l'une quelconque des revendications 9 à 10, dans lequel le rapport entre la force radiale et la force de tricotage est inférieur à environ 450.
